Europäisches Patentamt

European Patent Office

Office européen des brevets

(19)

(11) Publication number : **0 512 792 A1**

## (12) EUROPEAN PATENT APPLICATION

(21) Application number : 92304035.6

(22) Date of filing : 05.05.92

(51) Int. Cl.⁵ : **A61B 17/60, A61H 1/02**

(30) Priority : 06.05.91 US 696358
25.03.92

(43) Date of publication of application :
**11.11.92 Bulletin 92/46**

(84) Designated Contracting States :
**AT BE CH DE DK ES FR GB IT LI LU NL SE**

(71) Applicant : **SMITH & NEPHEW RICHARDS INC.**
**1450 Brooks Road**
**Memphis, Tennessee 38116 (US)**

(72) Inventor : **Hotchkiss, Robert N.**
**12718 Old Wick Road**
**San Antonio, Texas 78230 (US)**
Inventor : **Woodward, Arthur**
**2005 S.Kipling**
**Lakewood, Colorado 80227 (US)**
Inventor : **Hotchkiss, Kenneth W.**
**8808 Ute Drive**
**Golden, Colorado 80403 (US)**

(74) Representative : **Cole, William Gwyn, Dr.**
**Smith & Nephew p l c Corporate Patents and**
**Trade Marks Dept. Gilston Park**
**Harlow Essex CM20 2RQ (GB)**

(54) **Dynamic skeletal joint support.**

(57)   A dynamic skeletal joint support having proximal and distal support sections (113,115) and means (146) for rigidly connecting each support section to bone and a pair of hinges (117,119) connecting each support section to each other and pivoting at the joint (120) to cause movement of the support section and its corresponding attached bone through the movements of flexion and extension. The hinge may be driven in its movement by a gear mechanism (122,124) which may be disengaged by means of a clutch. The dynamic joint support may also include a distraction mechanism for movement of the bones out of contact in the joint, while allowing for an active range of motion at the joint.

FIG. 11.

EP 0 512 792 A1

## Field of the Invention

The present invention relates generally to the treatment of injuries and contractures of a skeletal joint and, more particularly, to a dynamic finger support for allowing the proximal interphalangeal (PIP) joint to be flexed and extended either by the patient actively or passively or by a continuous passive motion machine and maintain its alignment in the natural axis of rotation for managing contractures and joint instability.

## Background of the Invention

Flexion contractures or a tendency for muscles, tendons or scar tissue to shorten in skeletal joints are common after trauma and represent a major challenge in the care of such injuries. For example, a contracture of 30°-40° in the elbow can severely reduce upper extremity function.

Contracture and stiffness of the finger joints due to trauma, burns, or arthritis limits the overall function of the hand. motion of the proximal interphalangeal joint of the finger is especially important, as this joint accounts for more than 50% of the total active motion of the finger.

Current approaches to the treatment of joint trauma have more aggressively sought to prevent contracture and stiffness through movement. methods of rigid internal fixation with sufficient stability to allow motion within days after injury rather than closed treatment and immobilization in a cast have been developed. In the treatment of dislocations, protected early motion is now begun as soon as the patient is comfortably able to do so.

However, the currently available techniques for the prevention of contracture are not uniformly successful. Early active motion alone can reduce the severity of contracture, but requires the patient's own strength, compliance and constant effort and proper alignment and tracking of the joint cannot be insured. Passive stretching by a therapist can be done on a very limited basis and is applied slowly, but such therapy risks the formation of heterotopic bone and myositis ossifiactions. Passive stretching is not generally useful at the PIP joint, as it is painful and not uniformly successful. Dynamic splints may be used, but these require pressure on the sometimes sensitive or injured soft tissues, e.g. of the arm and forearm, and may not be possible, i.e. burn injury, or may reduce patient compliance. Examples of such splinting dynamic sling, polycentric cast brace hinges, or a hinged orthosis with rubber band traction.

Continuous passive motin (CPM) devices have been devloped which provide early motion gains, but these devices do not normally allow the joints to come to the extremes of motion which are the areas of greatest need. Further, these devices are not designed to insure accurate tracking or stability of the joint, but instead for example, in the elbow device, move the wrist relative to the shoulder or the humerous. These devices also rely on direct pressure on the soft tissues and skin, and thus are subject to the same limitations as the external splints discussed above.

Flexion-extension hinge distractors are hinged external fixators which are designed to hold the joints such as the elbow in distraction while permitting an active range of motion. These include the Volkov elbow hinge-distractor and the Deland and walker hinge distractor. These devices require the placement of a pin or wire into or in close proximity to kinematic axis of the elbow, with the pin acting as the mechanical axis of the device. Because these devices are difficult to align over the axis of rotation, pin tracking problems can occur. Furthermore, the mechanical axis cannot be realigned without reinsertion of the pin. In addition, these systems do not permit passive driving of the joint through a range of motion.

An additional problem associated with the flexion-fixation hinge distractors is the placement of pins in close proximity to the joint. Becuase of the movement of skin over and relative to underlying bone, movement of the skin in this area with normal flexion and extension of the joint in relation to a stationary pin can cause skin irritation and lead to infection. Such placement of the pins may also interfere with the treatment of a fracture by internal fixation.

Contracture and stiffness of the finger joints after trauma is currently prevented by immediate physical therapy of the involved digit. However, it is often difficult to achieve full active motion of the finger because soft tissue swelling and pain prevent the patient from maximal compliance during the critical first weeks of healing. If motion of the injured PIP joint is not initiated and maximised, scar formation around the joint becomes very strong. Later attempts to gain motion at the joint are then prevented by the strength of the scar tissue and the mechanical disadvantage of the passive splint or exercise program. Bony injury or joint instability often precludes actively or passively moving the digit through an effective range of motion, because motion risks redislocation or displacement of the skeletal elements on either side of the joint, or of the joint itself.

Because of the limitations with the currently available methods to prevent or treat joint injuries, patients often require surgical soft tissue release to improve the range of motion. Surgical release of contracture must be followed by many months of intensive therapy and splinting to maintain the gains in motion. Such maintenance is not uniformly successful, as the splinting and traditional therapies applied suffer from the same limitations as discussed above. moreover, oftentimes the cost of surgery and therapy, as well as the cost in time, lost wages and rehabilitation can be significant.

## Summary of the Invention

In order to solve the problems described above, a dynamic joint support is provided which includes proximal and distal external bracing sections, respectively connected to proximal and distal skeletal elements on opposite sides of a joint. The bracing sections are rigidly connected to their respective skeletal elements through wires or pins, e.g., in the dynamic finger support, through a clamping member which engages pins inserted into integral bone.

A hinge connects the bracing sections to each other in the vicinity of the joint so that the hinge can pivot at the joint when the skeeltal elements are moved through flexion or extension. The hinge includes an X-ray transparent material at the pivot point with a target mechanism, e.g., cross hairs, so the mechanical axis of the hinge can be aligned with the natural axis of the joint.

Appropriate adjusters are included with the external bracing sections for adjusting the length and orientation of the bracing sections relative to their respective skeletal elements and to the hinge for aligning the bracing sections relative to the joint. This alignment permits accurate placement of the axis of rotation of the device to recreate the normal kinematics of the joint.

The hinge also includes a gear mechanism which can be used for moving the bracing sections and consequently their respective skeletal elements relative to each other through the application of external force to the gear mechanism. The external force can be applied through a manually operated crank or a motor in order to stretch soft tissue surrounding the joint and thereby address a joint contracture. A clutch may also be provided so that the gear mechanism can be disengaged for allowing the skeletal elements to move freely under the patient's own muscle force through flexion and extension.

An adjustment mechanism can also be provided for placing the joint in distraction and maintaining its alignment in that position while the skeletal elements are moved through flexion and extension.

By providing the mechanism as described, contractures in the vicinity of a joint can be prevented through active or passive movement of the skeletal elements through the joint. Continuous passive motion can be applied to the joint with proper tracking and concomitant stability that comes from maintaining the skeletal elements in proper orientation relative to each other while they are being moved.

Further, the subject dynamic joint support also allows the joint to be held in distraction while permitting a range of motion without compression across the joint surfaces. All of the above can be performed without pins or wires in close proximity to the joint which eliminates skin irritation because there will be less skin motion relative to the underlying bone during movement. Further, the location of the pins is discretionary with the physician so that pins can be placed away from a fracture and not interfere with the healing process of a fracture in the vicinity of the joint. In addition, the apparatus may be designed to permit unrestricted access to the joint for medical and surgical procedures.

## Brief Description of the Drawings

A better understanding of the invention can be obtained from the detailed description of a preferred embodiment set forth below, when considered in conjunction with the appended drawings, in which:

Figure 1 is a top plan view of a dynamic finger support.

Figure 2 is a side plan view partially in section showing the attachment of the distal bracing section to the hinge.

Figure 3 is a bottom plan view partially in section showing the engagement of the proximal and distal hinge members.

Figure 4 is a side plan view of the dynamic finger support showing in dashed lines the pivotal engagement of the proximal and distal hinge members.

Figure 5 is a sectional view along line 5 of the Figure 2 showing the gear mechanism, gear shaft and finger dial.

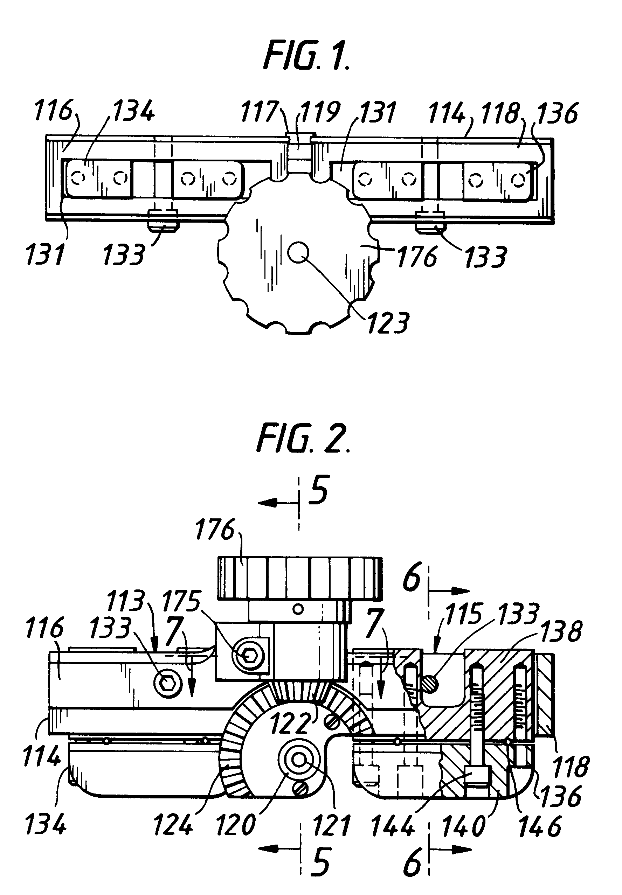

Finger 6 is a sectional view along line 6 of Figure 2 showing the engagement of the bracing section with the hinge member.

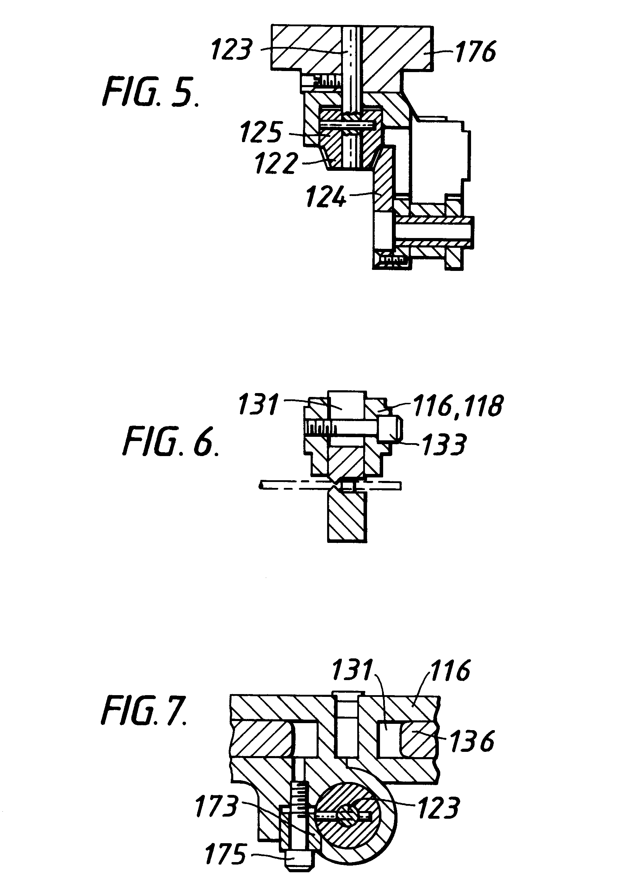

Figure 7 is a sectional view along line 7 of Figure 2 showing the gear means and locking means.

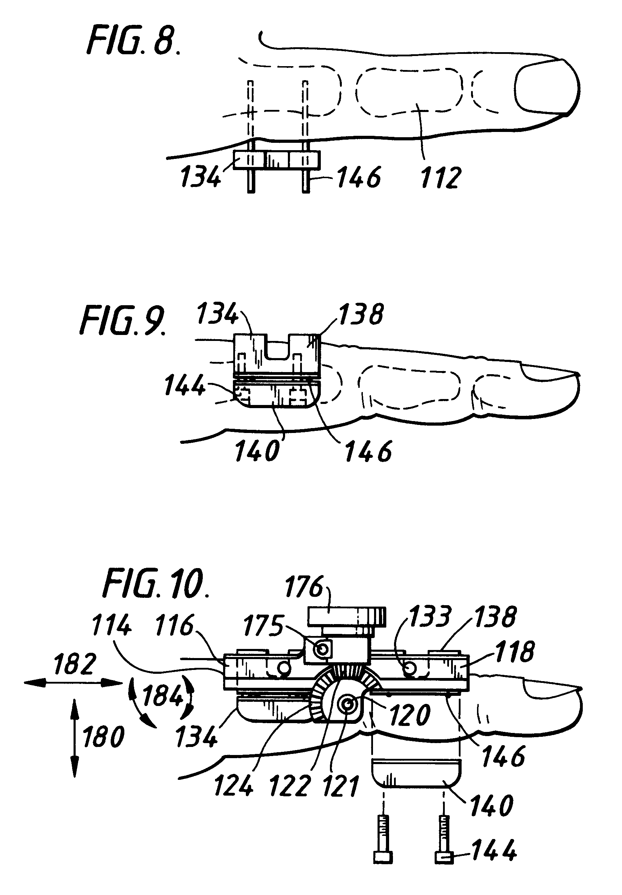

Figure 8 is a top plan view of the proximal bracing section as connected to pins in the proximal phalanx of a patent.

Figure 9 is a side plan view of the proximal bracing section as connected to pins in the proximal phalanx of a patient.

Figure 10 is a side plan view of the device as attached to the finger of a patent in extension.

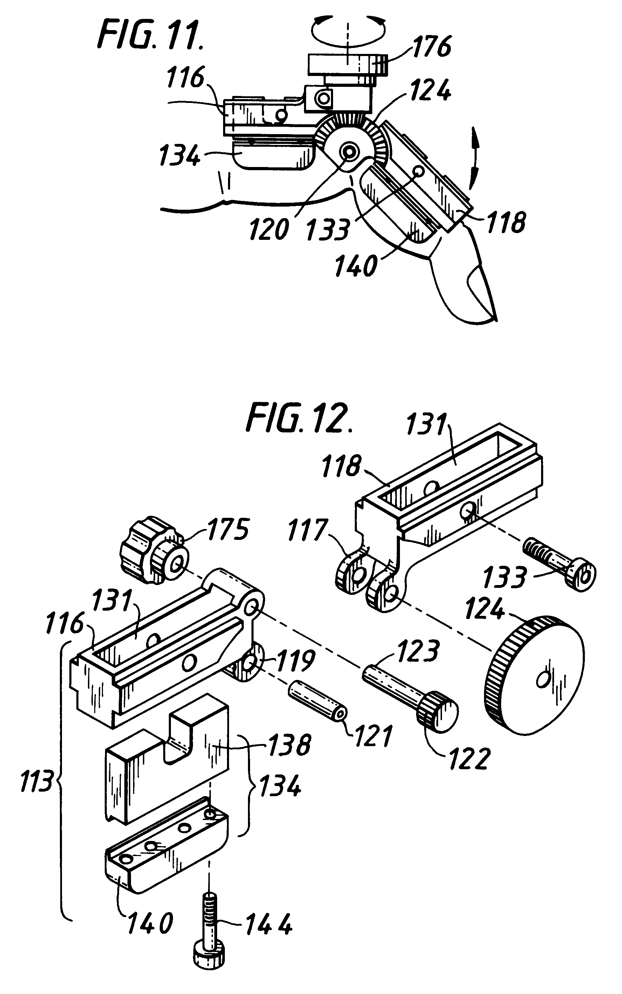

Figure 11 is a side plan view of the device as attached to the finger of a patent in flexion.

Figure 12 is an exploded view of the dynamic finger support.

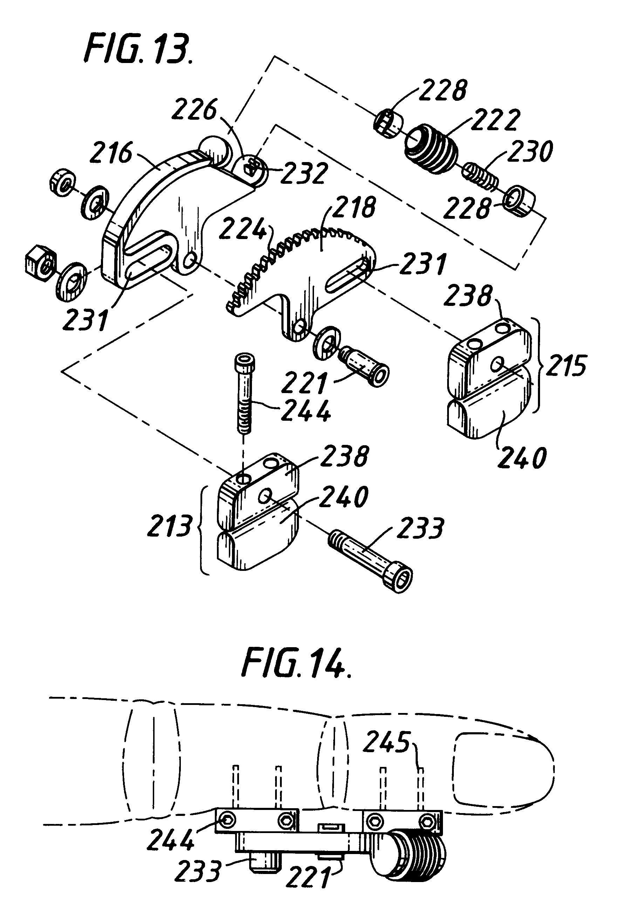

Figure 13 is an exploded view of a most preferred embodiment of the invention.

Figure 14 is a top plan view, partially in section, of the embodiment of Figure 13.

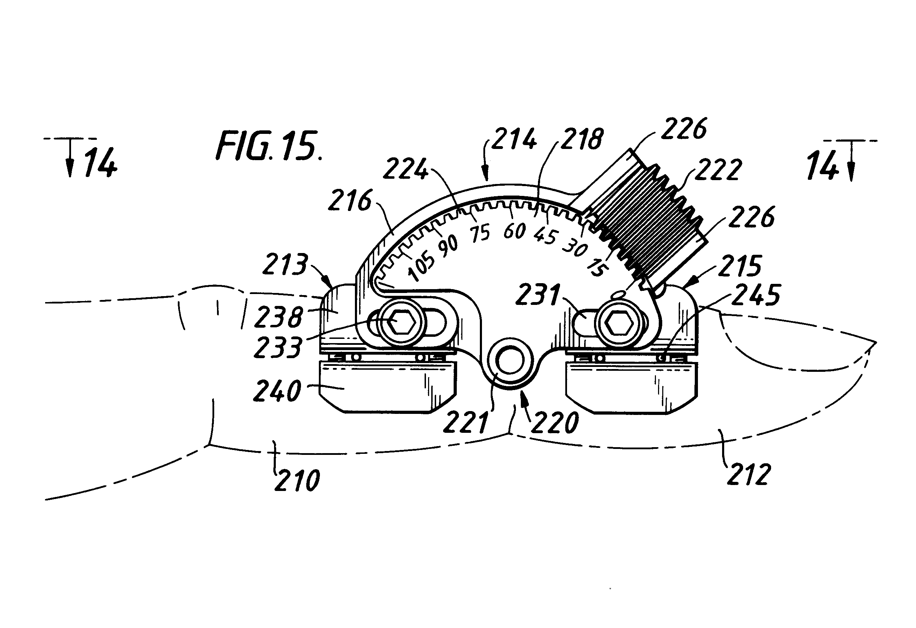

Figure 15 is a side plan view of the embodiment of Figure 13, showing the device in extension.

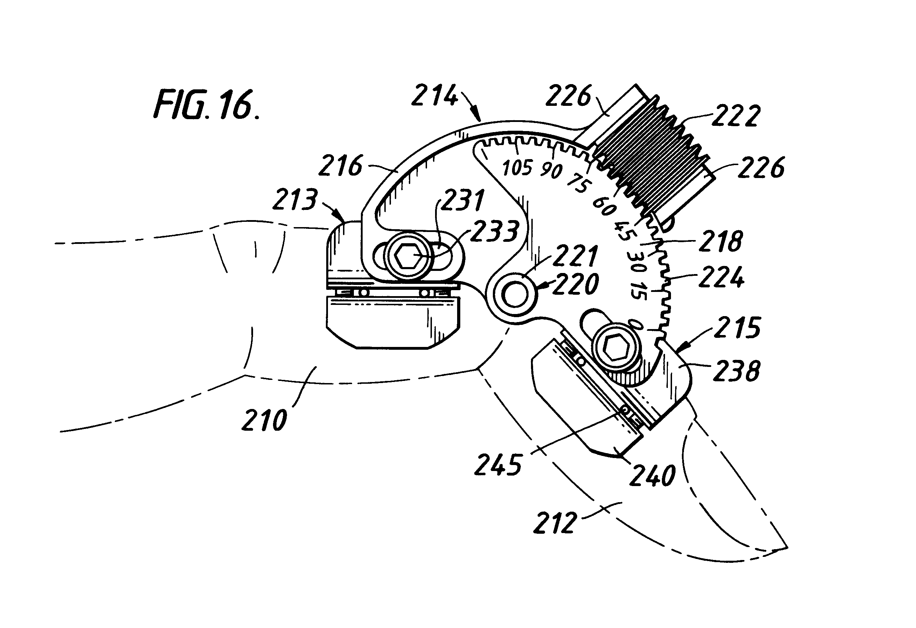

Figure 16 is a side plan view of the embodiment of Figure 13, showing the device in flexion.

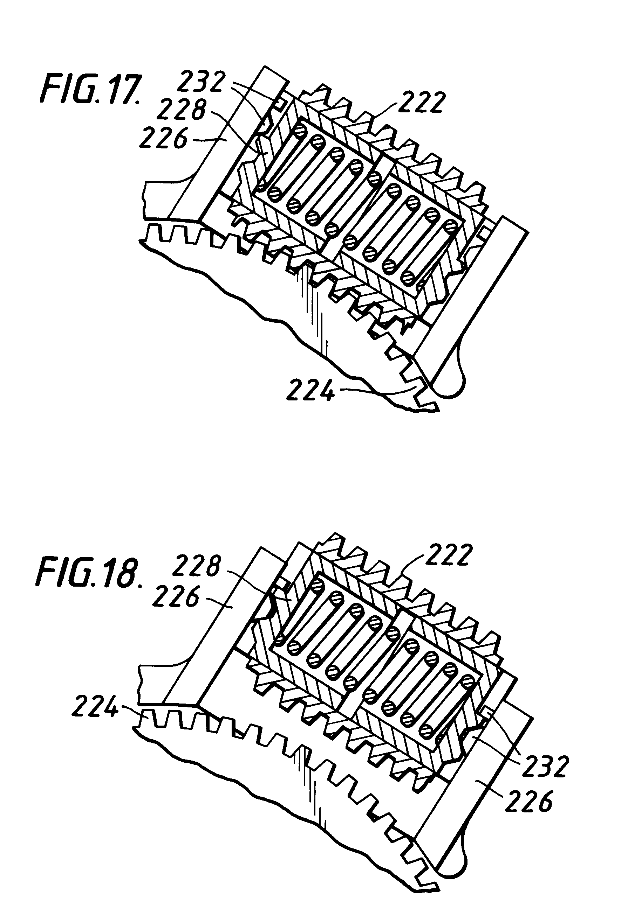

Figure 17 is a plan view partially in section showing the gear of the device in an engaged position.

Figure 18 is a plan view partially in section showing the gear of the device in a disengaged position.

## Detailed Description of the Invention

A dynamic joint support in a preferred embodiment of the present invention is shown as it would be connected to the finger of a patient in Figures 1 to 18, where reference numeral 110 identifies dashed lines illustrating the proximal phalanx of the patient's finger and 112 the distal phalanx. The finger support includes a hinge 114 which is collinear or aligned with the kinematic axis of the PIP joint. This alignment can be accomplished through the use of a X-ray machine, preferably in video (not shown) which can be used to slign the radiolucent hinge directly over the axis of the PIP joint, for example, using as a targeting mechanism a radiopaque axle.

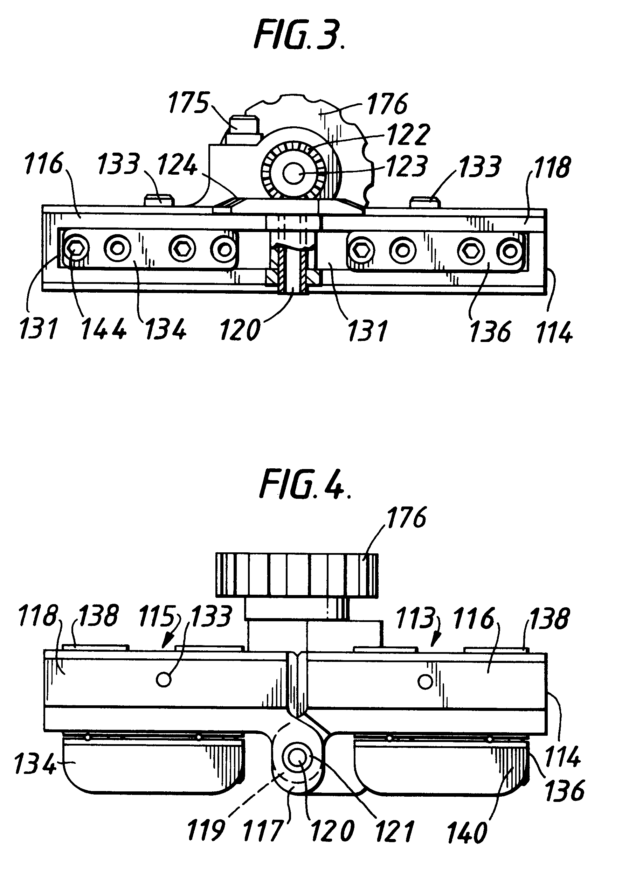

The dynamic finger support includes proximal and distal bracing sections 113, 115 which are adapted to rotate relative to one another, for example, as shown in Figure 4, by pivoting joint 120 formed between a receiving member 177 and extending member 119. A radiopaque axle 121 may serve as the axis of rotation of the hinge 114, and provide a target for alignment of the radiotranslucent finger hinge with the natural axis of the joint.

Each bracing section 113, 115 is adapted for attachment to pins 145 (see Figure 8) or wires (not shown) embedded in the proximal and distal phalanges 110, 112. For example, as shown in Figures 2, 9, and 28, each bracing section 113, 115 includes a hinge member 116, 118 adapted to receive a clamping member 134, 136. Each clamping member 134, 136 is formed with upper and lower jaws 138, 140, which are joined and tightened, for example, by vertical screws 144 on the engaged pins 146. One or more vertical screws 144 may be positioned in the clamping member away from the engaged pins 146. It is preferred that the communicating portions of the jaws 138, 140 be adapted to tightly clamp together and around the engaged pins, for example, by a fitted or tapered edge on each communication surface.

Each hinge section 116, 118 is adapted to receive its respective clamping member 134, 136. For example, as shown in Figures 17, 19, 22 and 28, clamping members 134, 136 engage hinge sections 116, 118 through a cavity 131 in each hinge section. Once inserted into the cavity 131, a fastener, e.g., horizontal screw 133 through the hinge section 116, 118 fixes the clamping member 134, 136 to the hinge sectin 116, 118.

A drive means is provided to move the proximal and distal bracing sections relative to each other and about the axle. For example, as shown best in Figure 2, the proximal hinge section 116 includes a first gear, e.g., a pinion 122 which mates with a second gear e.g., a fixed, curved rack 124 included in the distal hinge section 118. As shown in Figures 3 and 5, the first gear 122 is moved through rotation of a drive shaft 123 which translates rotational energy from the attached finger dial 176. When turned manually, the finger dial 176 will thus effect extension and flexion of the finger joint of the patient. As shown in Figures 18 and 28, the finger dial 176 and drive shaft 123 may be positioned perpendicular or normal to the axis of rotation.

As shown in Figures 2, 5 and 7 a locking means, e.g., set screw 175 can be provided for selectively locking or unlocking the relative position of the first and second gears. Tightening of the set screw 175 effects friction between a brake shoe 173 and the pinion 122 at a pinion collar 125, thereby causing a locked engagement of the gears. Releasing the gear screw 175 releases the friction and loosens the engagement and relative motion of the gears.

The elements of the hinge 114, as fixed onto the clamping members 134, 136 and the attached skeletal elements 110, 112 as described above, allow the PIP joint of the finger of a patient to move between the extended position of Figure 26, where the finger is relatively straight, and the flexed position of Figure 11, where the proximal phalanx 110 and distal phalanx 112 are moved toward each other. As described in detail below, the hinge 114 can be adjusted to permit precise alignment with the kinematic axis of the joint takes place, contractures are prevented, reduced or eliminated.

Referring to Figures 8-11, the installation of dynamic finger support of the present invention is shown. The proximal clamping member 134 is first positioned on one or more, preferably two or more, pins 146 inserted approximately along the lateral, mid-axial line of the proximal phalanx. The upper and lower jaws 138, 140 are positioned, respectively, posterior and anterior to the inserted pins 146, and secured to each other and the engaged pins by tightening of one or more vertical screws 114.

The hinge 114, including proximal and distal hinge members 116, 118 connected at pivoting joint 120, is positioned onto the proximal clamping member 134 by insertion of the proximal clamping member 134 into the cavity 131 of the proximal hinge member 116.

By X-ray analysis, preferably video, the axis of rotation of the hinge (axle) is aligned with the natural axis of rotation of the joint. Once the hinge is aligned with the axis of the joint, the relative position of the attached distal hinge member 118 is used as a guide to insert one or more pins into the distal phalanx. The distal clamping member 136 is then attached to the pins 145 and the attached distal clamping member 136 inserted into the cavity 131 of the distal hinge member 118, in a manner similar to that described above for the proximal counterparts.

In a most preferred embodiment, the dynamic finger support includes a unilateral hinge 214 having a first arcuate hinge member 216 and a second arcuate hinge member 218 rotatably connected to each other, for example, as shown in Figures 15 and 16 by a pivoting joint 220. A radiopaque axle 221 may serve as the axis of rotation of the hinge 214 and provide a indicia means for targeting alignment of the radiotranslucent finger hinge with the natural axis of the finger joint.

This most preferred embodiment is shown as it would be connected to the finger of a patient in Figures 15 and 16, where reference numeral 210 identifies dashed lines illustrating the proximal phalanx of the patient's finger and 212 the distal phalanx. The dynamic finger support includes proximal and distal external support sections 213, 215, each of which is adapted for attachment to pins 245 or wires (not shown) embedded in the skeletal elements of the proximal and distal phalanges 210, 212. For example, as shown in Figures 15 and 16, each support section 213, 215 includes superior and inferior clamping jaws 238, 240, which are joined and tightened, for example, by vertical screws 244 on the engaged pins 245. One or more vertical screws 244 may be positioned in the external support section away from the engaged pins 245.

Each arcuate hinge member 216, 218 is adapted to engage its respective external support section 213, 215. For example, as shown in Figures 13, 15 and 16, each arcuate hinge member 216, 218 is fastened to its respective external support Section 213, 215 by a fastener, e.g., horizontal screw 233 which traverses a slot 231 in the arcuate hinge member 216, 218 to engage and fasten the arcuate hinge member to its respective external support section 213, 215. Adjustments in a proximal-distal direction to the alignment of the hinge device with respect to the natural axis of the joint may be achieved by adjusting the arcuate hinge member proximally and/or distally with respect to the horizontal screw 233.

A gear means is provided to move the proximal and distal arcuate hinge members 216, 218 their attached external support sections, 213, 215, and thereby the attached proximal and distal skeletal elements relative to each other and about the axle 221. For example, as shown best in Figures 15 and 16, the proximal arcuate hinge member 216 includes a worm 222 which mates with teeth 224 on the distal arcuate hinge member 218. When rotated, the worm 222 translates rotational energy to the engaged teeth 224 and thus effects extension and flexion of the finger joint of the patient.

A clutch means is further provided for permitting selective engagement of the worm 222 and teeth 224, such that force is transferred between the worm 222 and teeth 24, restricting free motion of the skeletal elements and permitting controlled motion of the joint and for selectively disengaging the worm 222 and teeth 224 to permit the skeletal elements to move freely. As shown best in Figures 17 and 18, the worm 222 is adjustable within the worm housing 226 to alternative fixed positions, engaged (Figure 17) or disengaged (Figure 18). Preferably, the worm 222 includes endcaps 228 and a deformable spring 230 to facilitate movement of the worm 222 with respect to formations 232 on the engaging surface of the worm housing 226.

Referring to Figures 13 and 14, the installation of the dynamic finger support described above is shown. The proximal external support section 213 is first positioned on one or more, preferably two or more, pins 245 inserted approximately along the lateral, mid-axial line of the proximal phalanx. The superior and inferior clamping jaws 238, 240 are positioned, respectfully, posterior and anterior to the inserted pins 245, and secured to each other and the engaged pins by tightening of one or more vertical screws 244.

The hinge 214, including first and second arcuate hinge members 216, 218 connected at pivoting joint 220, is positioned first onto the proximal external support section 213 and secured thereto by horizontal screws 233.

By X-ray analysis, preferably video, the axis of rotation of the hinge as targeted by the radiopaque indicia, e.g., axle, is aligned with the natural axis of the joint. Once the hinge is aligned with the axis of the joint, the relative position of the attached second arcuate hinge member 218 is used as a guide to insert one or more pins into the distal phalanx. The distal external support section 215 is then attached to the pins 245 and the attached arcuate hinge member 218 is secured to the distal external support section 215 by horizontal screws 233.

Once the finger support is attached to the finger, adjustments may be made to further refine or correct the alignment of the axis of rotation of the hinge with the natural axis of the joint. The location of the clamping members 134, 136 within the hinge members 116, 118 may be adjusted in three planes, anterior-posterior (arrow 182), and tilted within the hinge members 116, 118 (arrow 184), i.e. to compensate for pin insertions which deviate from substantially parallel to the mid-axial line.

Distraction of the joint may be desired in treatment of injuries to the joint itself. Distraction of the PIP joint, for example, may be accomplished by manually pulling the digit in a distal direction prior to securing the hinge to the distal clamping member 136. While the joint is distracted in this manner, the distal hinge member 118 is secured to the distal clamping member 136. The attached hinge functions through its range of motion while the joint remains distracted.

It is contemplated that various sizes of the device will be preferable for various applications, e.g., when treating the finger of a child versus an adult, when treating the fourth digit versus the first.

Through the mechanism described, the dynamic finger support can be accurately aligned with the kinematic axis of the finger joint through the use of an X-ray machine, for example, by aligning a metal axle 121, or other radiopaque targeting device of the pivoting mechanical hinge 120, with the natural axis of the joint. Fine adjustments can be made by adjusting the position of the hinge members in the hinge members, as described above. Once the dynamic finger brace is accurately aligned in the preferred position, a patient can have his or her finger joint extended or flexed through the application of manual movement to the gearing mechanism. The dynamic joint brace of the present invention is useful in the treatment of trauma to a joint such as severe fractures, dislocations and the like where a high possibility of stiffness normally results from immobilization. The apparatus may be applied to the patient immediately to begin rehabilitation and prevent contracture. In some instances, it may be desirable to apply distraction to reduce the joint reaction force during flexion and extension. The dynamic brace allows for all of these treatments to occur through apparatus which is connected to bones on opposite sides of the joint and at a distance from the joint so as not to interfere with movement and rehabilitation.

The dynamic joint brace of the present invention also permits readjustments as needed during therapy without significant interference using external adjustment mechanisms. Adjustments may be necessary if the patient should fall or otherwise disturb the set alignment during therapy. Alternatively, the device permits monitored therapy, with immediate and easily accomplished adjustments in the proper alignment. Where appropriate, adjustments may be made in rotation, anterior-posterior positioning, and distal-proximal positioning of the hinge relative to the fixed skeletal elements.

The dynamic joint brace of the present invention may be fabricated using materials known in the field. It is important that the device be substantially radiotranslucent with a radio opaque targeting means at the axis of mechanical rotation to permit alignment of the axis of rotation of the hinge with the natural axis of rotation of the joint. It is preferred that the materials used to fabricate the device permit sterilization of the device. It is contemplated that portions of the device may be prepared from molds, i.e., the finger dial, shaft, and gear drive may be molded as one piece, or the rotating member may be molded as one piece including the half gear.

## Claims

1. A method for movably fixing a bone joint having a first bone section and a second bone section, the method comprising:
   stably fixing a first bracing section of a dynamic joint support to the first bone;
   stably fixing a second bracing section of the dynamic joint support to the second bone, the second bracing section pivotally connected to the first bracing section with pivot means having an axle alignable with an axis of rotation of the bone joint, wherein said fixing of said first and second bracing sections is to a bone section at a distance from the joint, and apart from the axis of rotation of the joint;
   adjusting to the joint support to align the axle with the axis of the bone joint; and
   applying force to a driving means on the joint support to drive the pivot means and thereby move the position of the first bone section with respect to the second bone section along the axis of the joint.

2. The method of claim 1, wherein said driving means comprises:
   cooperating first and second gears respectively included with the first and second bracing sections, the second gear adapted to move the first gear and thereby adjust the relative positions of the bracing sections and bone sections to which they are attached.

3. The method of claim 2, wherein
   the first gear is a curved rack having first teeth;
   the second gear is a pinion having second teeth adapted for engagement with the first teeth; and
   the seond gear having a finger dial connected thereto so that application of external force to the finger dial alters the relative position of the first and second gears and the first and second bracing sections and bone sections to which they are attached.

4. The method of claim 3, further including the step of:
   securing the gears against movement with respect to each other with a locking means to provide a friction hold of the first teeth with the second teeth.

5. The method of claim 4, wherein
   the second gear is connected to the finger dial by a drive shaft positioned within a housing on the second bracing section; and
   the locking means comprises a set screw adapted provide friction between the drive shaft and a collar encircling the drive shaft, and thereby prevent movement of the drive shaft.

6. The method of claim 1, wherein
   the bracing sections each include hinge member and a clamping member, each hinge member adapted to receive the clamping member, each clamping member adapted to receive and hold at least one pin, said at least one pin being inserted into a bone section of the joint.

7. The method of claim 6, wherein
the clamping members are movably secured within the hinge members.

8. The method of claim 7, wherein
the clamping members are movable in an anterior-posterior direction.

9. The method of claim 7, wherein
the clamping members are movable in a proximal-distal direction.

10. The method of claim 7, wherein
the clamping members are rotatable around the axis of the engaged pin.

11. The method of claim 1, wherein the pivot means comprises:
a first and second pivot member respectfully located at one end of each of the first and second bracing section, the first pivot member adapted to receive and be movably secured to the second pivot member, the axle passing through and securing together the pivot members.

12. The method of claim 1, including the step of:
aligning the axle of the pivot means with the axis of the joint using X-rays of the axle and the axis.

13. The method of claim 1, wherein the bone joint is a finger joint.

14. The method of claim 13, wherein the finger joint is an interphalangeal joint.

15. The method of claim 14, wherein the interphalangeal joint is a proximal interphalangeal joint.

16. The method of claim 15, wherein said fixing is achieved by attaching said bracing sections to pins inserted approximately into the lateral, mid-axial line of each of a proximal and a distal phalanx.

17. A method for movably fixing a bone joint having a first bone section and a second bone section, the method comprising:
stably fixing a first bracing section of a dynamic joint support to the first bone section;
stably fixing a second bracing section of the joint support to the second bone section, the second bracing section pivotally connected to the first bracing section with pivot means having an axle alignable with an axis of rotation the bone joint, wherein said fixing of said first and second bracing sections is to bone at a distance from the joint, and apart from the axis of rotation of the joint;
adjusting the joint support to align the axle with the axis of rotation of the bone joint;
applying force to a driving means to drive the pivot means and thereby move the position of the first bone segment with respect to the second bone segment along the axis of rotation of the joint,
wherein said drive means comprises cooperating first and second gears respectively included with the first and second bracing sections, the second gear adapted to move the first gear and thereby adjust the relative positions of the bracing sections and the bone segments to which they are attached; and
aligning the axle of the pivot means with the axis of rotation of the joint using X-rays of the axle and the axis.

18. A method for movably fixing a bone joint having a first bone section and a second bone section, the method comprising:
stably fixing a first bracing sectionn of a dynamic joint support to the first bone section;
stably fixing a second bracing section of the joint support to the second bone section, the second bracing section pivotally connected to the first bracing section with pivot means having an axle alignable with an axis of rotation of the bone joint, wherein said fixing of said first and second bracing sections is to bone at a distance from the joint, and apart from the axis of rotation of the joint, and the bracing sections each include a hinge member and a clamping member, each clamping member movably secured within each hinge member, and each clamping member adpated to receive and hold at least one pin, said at least one pin being inserted into bone;
aligning the axle of the pivot means with the axis of rotation of the joint using X-rays of the axle and the axis;
altering the relative position of the first and second bracing sections by the application of force to a cooperating first and second gears respectively included with the first and second bracing sections, the second gear having a finger dial connected thereto so that application of force to the finger dial alters the relative position of the first and second gears, the first and second bracing sections, and the bone sectins to which they are attached.

19. A dynamic joint support, comprising:
first bracing section;
second bracing section connected by pivot means to the first bracing section, wherein said first and second elements on opposite sides of a joint at a distance from the joint, and apart from an axis of rotation of the joint;

the pivot means including an axle at the axis of rotation for pivotally connecting and securing said first and second bracing sections; and

drive means for moving the first and second bracing sections relative to each other about the axle for relative movement of the skeletal elements.

20. The support of claim 1, wherein said bracing sections have interconnected therewith drive means for pivotally moving the first and second bracing sections relative to each other.

21. The support of claim 22, wherein said drive means comprises

cooperating first and second gears respectfully included with the first and second bracing sections, the second gear adapted to move the first gear and thereby adjust relative position of the support sections and the skeletal elements to which they are fixed.

22. The support of claim 23, wherein

the first gear is a curved rack having first teeth;

the second gear is a pinion having second teeth adapted for engagement with the first teeth; and

the second gear having a finger dial connected thereto so that application of external force to the finger dial alters the relative position of the first and second gears and thereby the first and second support sections and the skeletal elements to which they are fixed.

23. The joint support of claim 24, further comprising:

locking means for securing the gears against movement with respect to each other.

24. The joint support of claim 25, wherein

the second gear is connected to the finger dial by a drive shaft; and

the locking means comprises a set screw adapted to engage the drive shaft and thereby prevent movement thereof.

25. The joint support of claim 21, wherein the bracing sections each include a clamping member for receiving and holding at least one pin, said at least one pin being inserted into a skeletal element of the joint.

26. The joint support of claim 27, wherein

the clamping members are movably secured within the bracing sections.

27. The joint support of claim 28, wherein

the clamping members are movable in an anterior-posterior direction.

28. The joint support of claim 28, wherein

the clamping members are movable in a proximal-distal direction.

29. The joint support of claim 28, wherein

the clamping members are rotatable around the axis of the engaged pin.

30. The joint support of claim 21, wherein the pivot means comprises:

a first and second pivot member respectfully located at one end of each of the first and second bracing sections, wherein the first pivot member is adapted to receive and be movably secured to the second pivot member, the axle passing through and securing together the pivot members.

31. The joint support of claim 1, wherein the support is substantially radiotranslucent to permit X-ray alignment of the axle of the support with the axis of the joint.

32. The joint support of claim 33, further comprising a radiopaque target for aligning the axle of the support with the axis of the joint.

33. The joint support of claim 34, wherein the axle is radiopaque.

34. A dynamic joint support, comprising:

first bracing section;

second bracing section pivotally connected to the first bracing section, each bracing section adapted to be fixed to bone sections on opposite sides of a joint, each bracing section includes a clamping member movably secured within the bracing section for receiving and holding at least one pin, said at least one pin being inserted into a bone section at a distance from the joint and apart from the axis of rotation of the joint;

pivot means comprising a first and second pivot member respectfully located at one end of each of the first and second bracing sections and an axle, wherein the first pivot member is adapted to receive and be movably secured to the second pivot member, the axle passing through and securing together the pivot members;

radiopaque target for aligning the axle of the pivot means with the axis of the joint;

driving means including cooperating first and second gears respectfully attached to the first and second bracing sections, wherein the first gear is a curved rack having first teeth, the second gear is a pinion having second teeth adapted for engagement with the first teeth, and the second gear is connected to a finger dial by a drive shaft, so that application of external force to the finger dial is translated through the drive shaft to the gears to alter the relative position of the first and second gears, the first and second bracing sections and the bone sections to which they are fixed; and

locking means comprising a set screw adapted to optionally engage the drive shaft and thereby prevent movement thereof.

35. A dynamic finger support comprising:

proximal and distal external support sections, one for each skeletal element on opposite sides of a finger joint;

connecting means for rigidly connecting each support section to a bone on its respective side of the joint, wherein said connecting means attaches each support section to the bone at a distance from the joint and not at the axis of rotation of the joint;

hinge means having an axis for connecting the support sections to each other in the vicinity of the joint so the hinge means will pivot the joint when the skeletal elements move through flexion and extension, wherein said hinge means is a unilateral hinge including a first and second arcuate hinge member rotatably connected to each other, said first arcuate hinge member being non-movable and said second arcuate hinge member being adapted to move relative to the first arcuate hinge member; and

radiopaque indicia means for enabling alignment of the axis of the hinge with the axis of the joint.

36. The dynamic finger support of claim 35, further comprising:

adjustment means for adjusting the position and orientation of the hinge relative to the respective support sections and relative to the axis of the joint.

37. The dynamic joint support of claim 36, wherein the hinge means includes a gear means for moving the support sectinos and consequently their respective skeletal elements through flexion and extension in response to the applicatino of external force to the gear means.

38. The dynamic joint support of claim 37, wherein said gear means includes a worm movably mounted in said first arcuate hinge member, which worm mates with teeth located on said second arcuate hinge member, wherein rotation of the worm causes motion of the second arcuate hinge member relative to the first arcuate hinge member, thereby causing flexion or extension of the attached skeletal elements.

39. The dynamic joint support of claim 38, wherein the gear means further includes a clutch means for selectively engaging said gear such that force is transferred between said worm and said teeth to restrict free motion of the skeletal elements and permit controlled extension and flexion of the joint, and for selectively disengaging said worm and teeth to allow the skeletal elements to move freely.

40. A dynamic finger support comprising:

proximal and distal external support sections, one for each skeletal element on opposite sides of a finger joint;

connecting means for rigidly connecting each support section to a bone on its respective side of the joint, wherein said connecting means attaches each support section to bone at a distance from the joint and not at the axis of rotation of the joint;

hinge means having an axis for connecting the support sectins to each other in the vicinity of the joint so the hinge means will pivot the jiont when the skeletal elements move through flexion and extension,

wherein said hinge means is a unilateral hinge including a first and second arcuate hinge member rotatably connected to each other, said first arcuate hinge member being non-movable and said second arcuate hinge member being adapted to move relative to the first arcuate hinge member,

and wherein the hinge means includes a gear means including a worm movably mounted in said first arcuate hinge member, which worm mates with teeth located on said second arcuate hinge member, wherein rotation of the worm causes motion of the second arcuate hinge member relative to the first arcuate hinge member,

and wherein the gear means further includes a clutch means for selectively engaging said gear such that force is transferred between said worm and said teeth to restrict free motion of the skeletal elements and permit controlled extension and flexion of the joint, and for selectively disengaging said worm and teeth to allow the skeletal elements to move freely.

41. The dynamic finger support of claim 40, further comprising:

radiopaque indicia means for enabling alignment of the axis of the hinge with the axis of the joint.

42. The dynamic finger support of claim 41, further comprising:

adjustment means for adjusting the position and orientation of the hinge relative to the respective support sections and relative to the axis of the joint.

# FIG. 1.

# FIG. 2.

## FIG.3.

## FIG.4.

FIG. 5.

FIG. 6.

FIG. 7.

FIG. 8.

134    146    112

FIG. 9.    134    138

144

140    146

FIG. 10.    176

114    116    175    133    138

182    118

184    120    146

180    134

124  122  121    140

144

FIG.11.

FIG.12.

FIG.13.

FIG.14.

FIG.15.

FIG.16.

FIG.17.

FIG.18.

European Patent Office

**PARTIAL EUROPEAN SEARCH REPORT**

which under Rule 45 of the European Patent Convention shall be considered, for the purposes of subsequent proceedings, as the European search report

Application Number

EP 92 30 4035

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 612 919 (W.E. BEST) <br> * Column 3, lines 64-67; column 4, lines 3-8,44-62; figure 1 * | 19-23, 30 | A 61 B 17/60 <br> A 61 H 1/02 |
| Y | | 25-28, 31-33 | |
| X | THE JOURNAL OF BONE AND JOINT SURGERY, vol. 57-A, no. 5, July 1975, pages 591-600, Boston, US; M.V. VOLKOV et al.: "Restoration of function in the knee and elbow with a hinge-distractor apparatus" <br> * Page 591, column 2, lines 13-19; page 592, lines 14-18,29-48; figures 1-2 * | 19-21, 25-26, 28,30 | |
| Y | IDEM | 35-37 | |
| Y | FR-A-2 559 379 (ORTHOFIX) <br> * Page 1, lines 3-7; page 3, line 1 - page 5, line 12; figure 1 * <br> ---        -/- | 25-28 | |

| | |
|---|---|
| | TECHNICAL FIELDS SEARCHED (Int. Cl.5) |
| | A 61 B <br> A 61 H |

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely : 19-42

Claims searched incompletely :

Claims not searched : 1-18

Reason for the limitation of the search:

Method for treatment of the human or animal body by surgery or therapy (see Art. 52(4) of the European Patent Convention).

| Place of search | Date of completion of the search | Examiner |
|---|---|---|
| THE HAGUE | 28-07-1992 | NICE P.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0407)

European Patent Office

**PARTIAL EUROPEAN SEARCH REPORT**

Application Number

EP 92 30 4035

| | DOCUMENTS CONSIDERED TO BE RELEVANT | | CLASSIFICATION OF THE APPLICATION (Int. Cl. 5) |
|---|---|---|---|
| Category | Citation of document with indication, where appropriate, of relevant passages | Relevant to claim | |
| Y | FR-A-2 614 782 (ETABLISSEMENTS THUASNE) <br> * Page 6, lines 3-8,18-27 * <br> --- | 31-33, 35-37 | |
| A | GB-A-2 110 094 (P. HELLAND) <br> * Abstract; figure 1 * <br> --- | 38-40 | |
| P,X | EP-A-0 460 944 (SMITH & NEPHEW RICHARDS) <br> * Abstract; column 4, lines 21-26; page 5, columns 23-32; figure 5 * <br> ----- | 19-22, 25-33 | |
| | | | **TECHNICAL FIELDS SEARCHED (Int. Cl.5)** |

EPO FORM 1503 03.82 (P0410)